# FASCICULE DE BREVET EUROPEEN

(11) **EP 3 559 636 B1**
(45) Date de publication et mention de la délivrance du brevet: **19.07.2023**
(21) Numéro de dépôt: 17832524.7
(22) Date de dépôt: 21.12.2017
(51) Int. Cl.: G01N 21/47, G01N 21/51, G01N 15/06, G01N 15/14, C12Q 1/02, G01N 15/00, G01N 15/10

(54) **DISPOSITIF ET PROCÉDÉ POUR OBSERVER LE RAYONNEMENT RÉTRODIFFUSÉ PAR UN OBJET**
VORRICHTUNG UND VERFAHREN ZUR BEOBACHTUNG DER VON EINEM OBJEKT RÜCKGESTREUTEN STRAHLUNG
DEVICE AND METHOD FOR OBSERVING THE RADIATION BACKSCATTERED BY AN OBJECT

(30) Priorité: 26.12.2016 FR 1663397
(43) Date de publication de la demande: 30.10.2019
(73) Titulaire: Commissariat à l'Energie Atomique et aux Energies Alternatives, 75015 Paris (FR); Intelligence Artificielle Applications, 34060 Montpellier Cedex 2 (FR)
(72) Inventeur: SCHULTZ, Emmanuelle, 38120 Saint Egreve (FR); DECQ, Damien, 38000 Grenoble (FR); ROCH, Michel, 34670 Saint Bres (FR)
(74) Mandataire: INNOV-GROUP
(86) Numéro de dépôt international: PCT/FR2017/053769
(87) Numéro de publication internationale: WO 2018/122505

(56) Documents cités:
- EP-A1- 3 054 281
- WO-A1-2007/020554
- WO-A2-2016/054408
- US-A- 5 241 369
- US-A- 5 912 741
- KIM HUISUNG ET AL: "Reflected scatterometry for noninvasive interrogation of bacterial colonies", INTERNATIONAL SOCIETY FOR OPTICAL ENGINEERING, SPIE, PO BOX 10 BELLINGHAM WA 98227-0010 USA, vol. 21, no. 10, octobre 2016 (2016-10), page 107004, XP060075145, ISSN: 1083-3668, DOI: 10.1117/1.JBO.21.10.107004 [extrait le 2016-10-24]

## Description

### DOMAINE TECHNIQUE

Le domaine technique de l'invention est l'observation et l'identification d'un objet, notamment un objet biologique, en particulier une colonie bactérienne, sur la base d'une image d'un rayonnement rétrodiffusé par l'objet.

### ART ANTERIEUR

L'identification de microorganismes, et en particulier des bactéries, est un besoin concernant différents domaines. Dans le domaine du diagnostic, par exemple, l'identification de bactéries permet de connaître la nature d'agents pathogènes à l'origine d'une infection, et d'optimiser une prise en charge d'un patient. Par ailleurs, l'identification bactérienne est une technique de base dans l'épidémiologie, ou dans la lutte contre les infections nosocomiales. Au-delà de la santé, les applications peuvent concerner, de façon non exhaustive, l'hygiène, la sécurité, l'agroalimentaire.

On dispose actuellement d'une instrumentation performante et variée, permettant une telle identification. Les méthodes mises en oeuvre sont notamment la spectrométrie de masse, la spectrométrie Raman, des tests colorimétriques, l'analyse morphologique de colonies, ou des techniques d'amplification d'acides nucléiques. Les méthodes mettant en oeuvre une technique spectrométrique (spectrométrie de masse ou spectrométrie Raman) nécessitent un appareillage coûteux et réservé à des opérateurs qualifiés. Les méthodes colorimétriques sont plus simples, mais également plus lentes. En ce qui concerne l'amplification d'acides nucléiques, elle suppose un enchaînement de nombreuses étapes en respectant des conditions opératoires précises.

Le brevet US74665560 décrit une méthode permettant de caractériser un microorganisme basée sur l'exploitation de la diffusion et de la diffraction, par le microorganisme, d'un faisceau laser incident. Le microorganisme est disposé entre une source de lumière laser et un capteur d'image. Sous l'effet d'une illumination par le faisceau laser, on acquiert une image sur laquelle des figures de diffraction apparaissent, ces dernières constituant une signature du microorganisme observé. Le brevet US8787633 décrit une méthode répondant au même objectif. Ces documents décrivent une méthode d'identification bactérienne qui semble prometteuse, mais elle devient inapplicable dès lors que le milieu dans lequel sont disposées les bactéries est opaque, coloré ou diffusant. En effet, ces méthodes utilisent une image formée selon une configuration dite en transmission, dans laquelle l'échantillon est disposé entre une source de lumière et un capteur d'image. L'obtention d'une image exploitable est soumise à l'utilisation d'un échantillon suffisamment transparent. Ainsi, cette méthode n'est pas compatible avec des échantillons comportant un milieu de culture coloré, par exemple le milieu connu sous l'acronyme COS (Columbia Blood Ship) comportant une gélose columbia au sang de mouton. Elle n'est également pas applicable à un milieu diffusant de type CLED (Cystine Lactose Electrolyte Déficient), ou un milieu opaque de type gélose chocolat. Or, de tels milieux de culture sont très fréquemment (environ dans 70% des cas) utilisés dans le diagnostic clinique.

La demande de brevet WO2016/097063 adresse en partie ce problème, en proposant un procédé d'observation de microorganismes dans lequel on forme non pas une image selon une configuration en transmission, mais selon une configuration en rétrodiffusion. L'échantillon est illuminé par un faisceau laser. Le rayonnement rétrodiffusé est focalisé, par une optique de collection, sur un capteur d'image.

Le document US5241369 décrit un dispositif selon le préambule de la revendication 1, le dispositif permettant de former une image d'un rayonnement rétrodiffusé par un échantillon sur un écran translucide, ce dernier étant couplé à un capteur d'image. Selon ce dispositif, une source de lumière illumine un échantillon selon un angle d'incidence, de dernier étant incliné par rapport à l'axe optique du capteur d'image.

Le document WO2007/020554 décrit un dispositif d'observation d'un rayonnement diffusé par un échantillon recouvert d'un écran réfléchissant. A l'issue de multiples réflexions, l'image est focalisée sur un réflecteur convexe optiquement couplé à un capteur d'image. Une configuration similaire est décrite dans US5912741.

L'utilisation d'un écran translucide pour l'observation de la lumière diffusée par un objet a également été décrite dans la publication Huisung Kim et al "Reflected scatterometry for non invasive interrogation of bacterial colonies", International society for optical engineering, SPIE, vol. 21, N° 10, october 2016.

Les inventeurs ont mis en oeuvre un procédé proche de celui décrit dans WO2016/097063 et ont constaté certaines limitations, décrites ci-après. L'objectif de l'invention est de surmonter ces limitations, en proposant un procédé d'observation et de caractérisation de microorganismes selon une configuration en rétrodiffusion. L'invention est particulièrement adaptée à un échantillon opaque, tout en restant naturellement applicable à des échantillons transparents. Elle permet une observation et une caractérisation de colonies de microorganismes à différents stades de développement, qu'il s'agisse de microcolonies ou de macrocolonies. Un autre avantage est qu'elle est simple à mettre en oeuvre et robuste, et ne requiert une instrumentation coûteuse. Par ailleurs, le procédé mis en oeuvre est non destructif. Il peut être appliqué sur une colonie, dans son milieu de culture, sans nécessiter de prélèvement. Enfin, l'analyse est rapide, de l'ordre de la seconde.

### EXPOSE DE L'INVENTION

L'invention concerne tout d'abord un dispositif selon la revendication 1.

La source de lumière peut notamment être une source de lumière laser. Le dispositif peut comporter une optique de collimation, de manière à ce que le faisceau lumineux émis par la source de lumière soit collimaté. Le dispositif peut comporter une optique d'expansion de faisceau, de manière à ajuster le diamètre du faisceau laser à la taille et à la morphologie de l'objet analysé.

L'objet peut être une colonie de microorganismes, par exemple une colonie bactérienne, auquel cas l'écran permet l'obtention d'un diffractogramme dont la taille est suffisamment importante pour caractériser une colonie à un stade de développement suffisamment avancé.

Le dispositif peut comporter l'une quelconque des caractéristiques suivantes, prises isolément ou selon les combinaisons techniquement réalisables :
- L'aire de la première face de l'écran est supérieure à 100 cm².
- Le diamètre de l'ouverture est inférieur à 2 cm.
- La surface de l'ouverture est inférieure à 1 cm² ou à 2 cm².
- L'écran a une forme courbée, notamment en s'incurvant vers l'échantillon.
- L'écran comporte un guide de lumière pour transporter une lumière entre la première face et la deuxième face. L'écran peut notamment comporter plusieurs fibres optiques s'étendant entre la première face et la deuxième face.
- L'écran transmet moins de 90% du rayonnement rétrodiffusé de la première face vers la deuxième face.
- Le support est mobile par rapport à l'écran, la distance entre le support et l'écran pouvant être ajustée.
- Le dispositif comporte un capteur d'image dit auxiliaire, le capteur d'image auxiliaire étant configuré pour acquérir une image, dite image auxiliaire, du diffractogramme formé sur l'écran, l'écran étant disposé entre le capteur d'image auxiliaire et l'objet ; le dispositif comporte également un processeur configuré pour combiner l'image acquise par le capteur d'image et l'image auxiliaire acquise par le capteur d'image auxiliaire pour former une image représentative du diffractogramme.
- Le capteur d'image est apte à occuper différentes positions, et d'acquérir une image du diffractogramme, formé sur l'écran, dans chacune des positions, le dispositif comportant également un processeur, apte à traiter l'image acquise par le capteur d'image dans chaque position pour former une image représentative du diffractogramme.
- Le faisceau incident se propage entre l'écran et l'objet autour d'un axe dit axe d'incidence, le dispositif comportant un réflecteur dit annulaire, s'étendant autour de l'axe d'incidence, entre l'échantillon et l'écran, le réflecteur annulaire étant apte à réfléchir une partie du rayonnement rétrodiffusé vers l'écran.
- Le capteur d'image peut être centré par rapport à l'axe d'incidence selon lequel se propage le faisceau incident atteignant l'objet (ou de l'axe de rétrodiffusion autour duquel se propage le rayonnement de rétrodiffusion).

Un autre objet de l'invention est un procédé d'observation selon la revendication 9.

Le procédé peut comprendre l'une quelconque des caractéristiques décrites ci-après, prises isolément ou selon les combinaisons techniquement réalisables :
- La surface de l'ouverture est inférieure à 2 cm², ou à 1 cm².
- La surface de la première face de l'écran est supérieure à 100 cm².
- L'écran est incurvé, en s'incurvant notamment vers l'échantillon.
- L'écran agit alors en tant que rétro-écran, en transmettant le diffractogramme, projeté sur la première face, vers la deuxième face.
- L'écran est translucide.
- L'écran comporte au moins un guide de lumière, notamment une fibre optique, s'étendant entre la première face et la deuxième face.
- Une face de l'écran est structurée de façon à former une lentille.
- L'écran transmet moins de 90% du rayonnement rétrodiffusé.
- Le procédé comporte, suite à l'étape c), une étape d'ajustement de la distance entre l'échantillon et l'écran en fonction de l'image acquise par le capteur d'image, les étapes a) à c) étant répétées après l'ajustement de ladite distance.
- L'étape c) comporte l'acquisition de plusieurs images de l'écran, chaque image étant acquise à partir d'une position différente par rapport à l'écran, ainsi qu'une étape de combinaison des images ainsi acquises pour établir une image, dite image résultante, du diffractogramme formé sur l'écran.
- Le procédé comporte une étape d) de caractérisation de l'objet à partir de l'image acquise par le capteur d'image, ou à partir de l'image résultante. La caractérisation de l'image peut comporter :
   · une détermination de caractéristiques de l'image ;
   · une identification de l'objet à l'aide desdites caractéristiques et de caractéristiques de calibration établies en mettant en oeuvre les étapes a) à c) du procédé à l'aide d'un échantillon étalon.
- L'objet comporte un microorganisme. L'objet peut notamment regrouper plusieurs microorganismes formant une colonie. L'objet peut être une colonie bactérienne.
- Le procédé est mis en oeuvre avec un dispositif tel que décrit dans la demande.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

### FIGURES

La figure 1A représente un dispositif d'observation de microorganismes selon l'art antérieur.
Les figures 1B et 1C illustrent des distributions spatiales de rayonnements de rétrodiffusion émanant respectivement de deux objets différents.
Les figures 2A, 2B représentent un exemple qui n'est pas conforme à l'invention. La figure 2C montre un exemple d'écran pouvant être mis en oeuvre dans le premier, le deuxième ou le troisième mode de réalisation. La figure 2D est un détail du faisceau incident à l'échantillon et du rayonnement rétrodiffusé par l'échantillon. Les figures 2E et 2F illustrent une variation de la distance entre l'écran et l'échantillon. La figure 2G est un exemple de système optique de mise en forme du faisceau laser.
Les figures 3A et 3B illustrent une variante. La figure 3C représente une
Les figures 4A et 4B schématisent un mode de réalisation.
Les figures 5A, 5B, 5C et 5D représentent des diffractogrammes obtenus en mettant en oeuvre un mode de réalisation de l'invention.
Les figures 6A et 6B montrent des diffractogrammes obtenus en mettant en oeuvre un autre mode de réalisation de l'invention.
Les figures 7A et 7B montrent des diffractogrammes obtenus en mettant respectivement en oeuvre deux configurations différentes.
Les figures 8A et 8B représentent une configuration mise en oeuvre lors d'essais expérimentaux.
Les figures 8C et 8D sont des diffractogrammes d'une colonie de bactéries respectivement obtenus en utilisant la configuration représentée sur la figure 8A, avec un écran incurvé, et une configuration telle que représentée sur la figure 4B, avec un écran plan.
Les figures 8E et 8F sont des diffractogrammes d'une colonie de bactéries respectivement obtenus en utilisant la configuration représentée sur la figure 8B, avec un écran incurvé, et une configuration telle que représentée sur la figure 4B, avec un écran plan.
La figure 8G est un diffractogramme d'une colonie de bactéries obtenu en utilisant la configuration représentée sur la figure 8B.

### EXPOSE DE MODES DE REALISATION PARTICULIERS

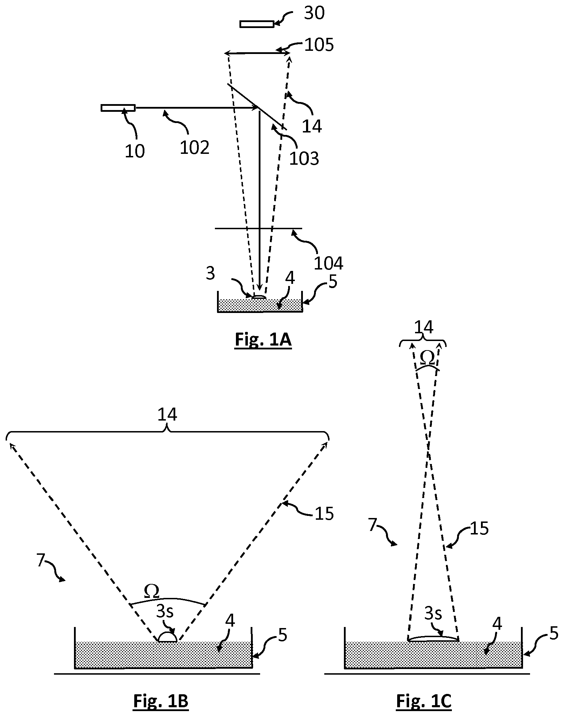

On a représenté, sur la figure 1A, un dispositif d'observation de microorganismes tel que décrit dans la demande de brevet WO2016/097063. Une source de lumière laser 10 émet un faisceau lumineux polarisé rectilignement 102 se propageant jusqu'à un objet 3 à caractériser, par exemple une colonie bactérienne disposée à la surface d'un milieu de culture 4. Avant d'atteindre la colonie bactérienne 3, le faisceau lumineux polarisé 102 est dévié par une lame semi-réfléchissante 103, de façon à se propager selon une direction, dite direction d'incidence, sensiblement perpendiculaire à la surface du milieu de culture 4. Le faisceau lumineux 102 traverse une lame quart d'onde 104 avant d'atteindre la colonie bactérienne 3. Le faisceau lumineux 102 interagit avec la colonie bactérienne 3, ce qui résulte en la formation d'un rayonnement rétrodiffusé 14 se propageant selon une direction sensiblement opposée à la direction d'incidence. Le rayonnement rétrodiffusé 14 est formé par de multiples interactions du faisceau lumineux 102 avec la colonie 3, combinant les effets de diffraction et de diffusion élastique dans la colonie. Le rayonnement rétrodiffusé 14 traverse la lame quart d'onde 104, puis la lame semi-réfléchissante 103, avant d'être focalisé par un système optique 107 vers un capteur d'image 30. L'image formée sur le capteur d'image, désignée par le terme diffractogramme, est représentative du rayonnement de rétrodiffusion 14. Le diffractogramme peut constituer une signature de la colonie bactérienne, permettant une identification des bactéries formant la colonie. Ce dispositif, représentant l'art antérieur, a été mis en oeuvre par les inventeurs. Ces derniers ont montré que ce dispositif ne permettait pas une observation satisfaisante de certaines colonies bactériennes.

En effet, le rayonnement rétrodiffusé 14 est émis selon une plage angulaire variable en fonction du type de microorganisme observé. Certaines colonies bactériennes, par exemple une colonie de staphylocoques, se développent en formant progressivement une surface externe 3s ayant une forme proche d'un hémisphère délimité par un milieu ambiant 7, par exemple de l'air. Un tel cas de figure est illustré sur la figure 1B. Dans ce type de configuration, le rayonnement rétrodiffusé 14 se propage de façon divergente, en formant un cône 15 selon une plage angulaire Ω élevée. Par élevée, on entend comprenant des angles supérieurs à 65° voire à 85°. Cela est notamment dû à la réfraction du rayonnement rétrodiffusé lorsqu'il franchit la surface 3s pour être réfracté dans le milieu ambiant 7. A l'inverse, comme représenté sur la figure 1C, d'autres colonies bactériennes se développent en formant progressivement une surface 3s plane, dont le rayon de courbure est élevé. De ce fait, le rayonnement rétrodiffusé 14 est réfracté dans le milieu ambiant 7 et se propage dans ce dernier selon un faisceau convergent, formant un cône 15, d'angle au sommet Ω. Les bactéries de type *enterobacteriace* forment des colonies présentant une telle morphologie. Ainsi, en fonction du type des microorganismes observés et de leur stade de développement, la morphologie d'une colonie évolue, conditionnant la distribution spatiale du rayonnement rétrodiffusé 14. Une limite du dispositif décrit dans WO2016/097063 est que le champ d'observation est faible et fixe, ne convenant pas aux colonies bactériennes dont la forme se rapproche de l'exemple de la figure 1B. Les inventeurs ont conçu un dispositif d'observation tenant compte de la variabilité de la distribution spatiale du rayonnement rétrodiffusé 14. Plus précisément, le dispositif, selon l'invention, dispose d'un champ d'observation pouvant s'adapter au microorganisme observé. En effet, les inventeurs ont établi que pour les microorganismes générant un rayonnement rétrodiffusé dont la distribution spatiale est illustrée sur la figure 1B, un champ d'observation très large peut être nécessaire.

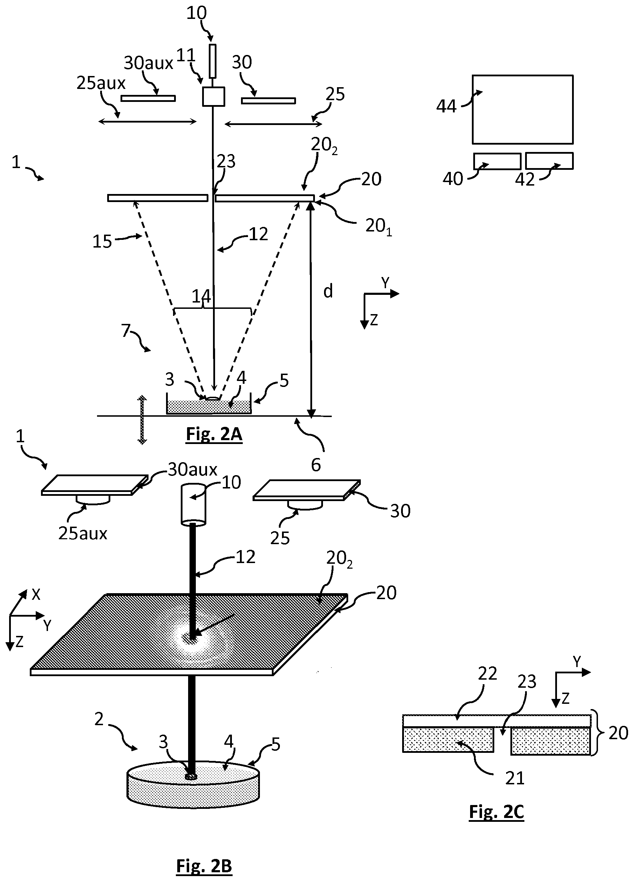

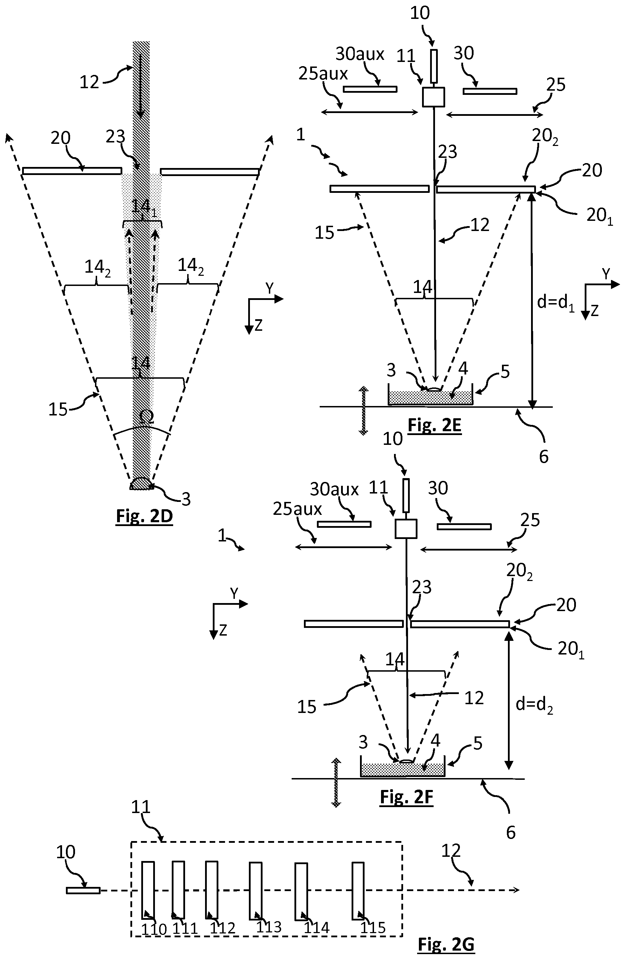

La figure 2A représente un premier example d'un dispositif 1 qui n'est pas conforme à l'invention. Le dispositif comporte une source de lumière 10, apte à émettre un faisceau lumineux 12, dit faisceau incident, se propageant jusqu'à un échantillon 2 comportant un objet 3 à caractériser. La source de lumière 10 est de préférence temporellement et spatialement cohérente. La source de lumière 10 est de préférence une source laser. Selon une variante, la source de lumière peut être une diode électroluminescente ou une source de lumière blanche. Il est alors préférable que la source de lumière soit suffisamment ponctuelle pour être spatialement cohérente. Cela peut être obtenu en associant la source de lumière 10 à un filtre spatial, par exemple un diaphragme, ou à une fibre optique. La source de lumière 10 peut également être associée à un filtre passe-bande, de manière à obtenir une bande spectrale d'émission Δλ suffisamment étroite, et de préférence inférieure à 50 nm voire 10 nm. La source de lumière est avantageusement couplée à un système de mise en forme du faisceau laser 11, décrit par la suite en lien avec la figure 2G.

Le faisceau incident 12 émis par la source de lumière et se propageant vers l'objet 3 est de préférence un faisceau parallèle, dont le diamètre peut avantageusement être ajusté. Le diamètre du faisceau incident 12 est de préférence compris entre 100 µm et 10 mm. L'ajustement du diamètre permet de s'adapter à la taille de l'objet 3 à caractériser. Ainsi, lorsque l'objet 3 est une colonie bactérienne, cela permet d'ajuster la taille du faisceau incident 12 à la morphologie de la colonie, cette dernière dépendant du type de bactérie et du stade de développement. Un système optique de mise en forme 11 peut être disposé entre la source de lumière 10 et l'objet 3. Un exemple de système optique de mise en forme 11 est donné en lien avec la figure 2G. Le système optique de mise en forme 11 peut permettre un ajustement du diamètre du faisceau incident 12 par rapport à l'objet 3, notamment sa taille ou sa morphologie. Il peut également uniformiser une distribution spatiale de l'énergie dans le faisceau incident 12, de façon que l'intensité lumineuse soit plus homogène dans le faisceau. Le faisceau incident 12 se propage vers l'objet à travers une ouverture 23 ménagée dans un écran 20, dont la fonction est décrite ultérieurement. Le diamètre de l'ouverture 23 est de préférence inférieur à 2 cm, et est par exemple inférieur à 1 cm. De préférence, le diamètre de l'ouverture 23 est ajusté de façon à être supérieur au diamètre du faisceau incident 12, en étant le plus proche possible de ce dernier. La source de lumière 10 est disposée de façon à émettre un faisceau incident 12 se propageant vers l'objet 3 et atteignant ce dernier selon une incidence orthogonale ou sensiblement orthogonale.

L'objet à caractériser 3 peut être un microorganisme ou un ensemble de microorganismes formant une colonie. Le microorganisme peut être une bactérie, une levure, un champignon ou une microalgue. L'objet à caractériser peut également être un groupe de cellules, formant par exemple un amas. L'objet à caractériser peut être en contact avec un milieu de culture 4, en étant disposé dans ce dernier ou à la surface de ce dernier. Le milieu de culture 4 est confiné dans une enceinte 5. Le milieu de culture 4 et/ou l'enceinte 5 peut être opaque ou translucide.

En particulier, il n'est pas nécessaire que le milieu de culture 4 et l'enceinte 5 soient transparents, condition imposée par les procédés basés sur des configurations en transmission décrites en lien avec l'art antérieur. L'ensemble formé par l'enceinte 5, le milieu de culture 4 et l'objet 3 constitue l'échantillon 2, ce dernier reposant sur un support 6. Dans l'exemple représenté, le support est une platine plane mobile en translation selon un axe Z, dit axe d'incidence. L'invention est particulièrement adaptée aux échantillons comportant un milieu de culture 4 opaque. Lorsque le milieu de culture 4 n'est pas suffisamment opaque, il est préférable que l'enceinte 5 soit opaque, et de préférence absorbante, de façon à minimiser des réflexions parasites. L'enceinte 5 peut comporter un couvercle, sous réserve que ce dernier soit transparent. Lorsque l'enceinte 5 est transparente, il est préférable qu'elle soit disposée sur un support 6 opaque ou translucide. Un tel support évite les réflexions parasites.

Sous l'effet de l'illumination par le faisceau incident 12, l'objet 3 émet un rayonnement rétrodiffusé 14, se propageant selon ou autour d'un axe central de rétropropagation -Z parallèle à l'axe d'incidence Z, et de sens opposé à ce dernier. D'une façon générale, le terme rayonnement rétrodiffusé désigne un rayonnement se propageant selon un axe de propagation comportant une composante opposée à l'axe d'incidence Z. Le rayonnement rétrodiffusé 14 résulte de l'interaction des photons du faisceau incident 12 avec l'objet 3, ce dernier présentant un indice de réfraction plus élevé que l'indice de réfraction du milieu ambiant 7 dans lequel se propage le faisceau incident, le milieu ambiant 7 étant généralement de l'air. Du fait de l'angle d'incidence, la majeure partie du faisceau incident 12 pénètre dans l'objet 3 en formant un faisceau incident réfracté. Le faisceau incident 12 réfracté dans l'objet 3 subit une ou plusieurs diffusions élastiques dans l'objet, et peut générer des ondes de diffraction. Un rayonnement rétrodiffusé 14 émane de l'objet en se propageant à travers la surface 3s, comme décrit en lien avec les figures 1B et 1C. Le rayonnement rétrodiffusé est réfracté dans le milieu ambiant 7, puis se propage, autour de l'axe de rétropropagation -Z, jusqu'à un écran 20, sur lequel il forme une image I₂₀, dite diffractogramme, représentative du rayonnement de rétrodiffusion. L'axe de rétropropagation est coaxial de l'axe d'incidence. Dans la littérature anglosaxonne, le diffractogramme est usuellement désigné par le terme "scattergram", ou "scattering pattern", ou « scatterograph », que l'on pourrait également traduire par le terme diffusiogramme. On note qu'aucune optique de focalisation ou de formation d'image n'est disposée entre l'objet 3 et l'écran 20.

L'écran 20 est apte à collecter le rayonnement 14 rétrodiffusé par l'objet 3 lorsqu'il est illuminé par le faisceau lumineux 12. Le terme écran désigne un élément dont une première face 20₁ collecte le rayonnement rétrodiffusé 14, ce dernier étant projeté sur ladite première face 20₁. Ainsi, le diffractogramme I₂₀ se forme sur la première face 20₁ de l'écran 20. L'écran 20 s'entend selon le plan de l'échantillon XY, selon une aire d'au moins 50 cm², mais il est préférable que l'aire soit supérieure à 100 cm², voire supérieure à 200 cm², et par exemple de 400 cm², soit un carré de 20 cm de côté. Le faisceau incident 12 est de préférence centré par rapport à l'objet 3, selon le plan de l'échantillon XY.

Le dispositif comporte au moins un capteur d'image 30, pour acquérir une image I₃₀ du diffractogramme I₂₀ formé sur l'écran. Le capteur d'image 30 peut notamment être un capteur matriciel comportant des pixels arrangés selon une matrice, chaque pixel formant un photodétecteur élémentaire. Le capteur d'image 30 est par exemple un capteur de type CCD ou CMOS. Le capteur d'image 30 est relié à un processeur 40, par exemple un microprocesseur, comportant une mémoire 42 dans laquelle sont stockées des instructions de traitement d'image, permettant l'analyse de l'image acquise par le capteur d'image 30 en vue de caractériser l'objet 3. Le processeur 40 peut également permettre un déplacement du support 6 par rapport à l'écran 20, comme expliqué ci-après. Un moniteur 44 permet de visualiser l'image acquise.

Afin de permettre une projection du diffractogramme sur l'écran 20, l'écran 20 n'est pas totalement transparent : il interagit avec le rayonnement diffusé 14, par absorption et/ou diffusion. De préférence, l'écran transmet jusqu'à 80%, voire 90% ou 95% du rayonnement rétrodiffusé, la partie non transmise étant absorbée ou diffusée. Les inventeurs estiment qu'une transmittance de l'ordre de 75% est optimale. Par transmittance, on entend un ratio entre une intensité d'un rayonnement transmis par l'écran et une intensité d'un rayonnement incident à l'écran. La transmittance de l'écran est de préférence inférieure à 95%, voire inférieure à 90% ou 80%. L'opacité est définie comme étant l'inverse de la transmittance. L'écran 20 comporte une deuxième face 20₂, s'étendant, de préférence, parallèlement à la première face 20₁. L'écran 20 est configuré de telle sorte que l'image projetée sur la première face 20₁, en l'occurrence le diffractogramme, apparaisse également, par transparence et/ou par diffusion, sur la deuxième face 20₂. L'écran 20 fonctionne alors selon un rétroécran, ou écran de rétroprojection, en étant interposé entre la source du rayonnement diffusé, en l'occurrence l'objet 3, et le capteur d'image 30. L'écran 20 peut être translucide, le terme translucide désignant un matériau non transparent, mais laissant passer la lumière, et à travers lequel des éléments apparaissent flous. Il s'agit par exemple d'un substrat de papier calque, d'un substrat comportant des éléments diffusants, par exemple des microbilles, ou encore d'une toile ou d'une plaque de verre dépoli. Lorsque l'écran comporte des microbilles, il peut s'agir de microbilles en polycarbonate. Des écrans de rétroprojection, sous la forme de toiles, convenant à cette application sont par exemple fournis par la société Multivision, sous les références "retro gris" ou "retro crème". Lorsque l'écran 20 est un papier calque, il peut comporter une surface rugueuse dont la rugosité Bendtsen atteint 100 à 300 ml/mm, la rugosité Bendtsen étant déterminée selon la norme NF 8791-2. Le diffractogramme formé sur la première face 20₁ apparaît sur la deuxième face 20₂, comme représenté sur la figure 2B.

Le dispositif comporte une optique de focalisation 25, permettant de focaliser le diffractogramme I₂₀ formé sur la deuxième face 20₂ de l'écran 20, de telle sorte que l'image I₃₀ acquise par le capteur d'image 30 corresponde à ce diffractogramme. De préférence, le capteur d'image 30 s'étend parallèlement à l'écran 20, et l'optique de focalisation 25 comporte un axe optique coaxial de l'axe de rétropropagation -Z (ou de l'axe d'incidence Z). Cependant, dans ce mode de réalisation, le capteur d'image 30 n'est pas centré par rapport à l'axe de rétropropagation. Aussi, sur l'image acquise par le capteur d'image 30, le diffractogramme peut être déformé. Une telle déformation peut être corrigée par des algorithmes basés sur une calibration, prenant en compte la position du capteur d'image 30, et de son éventuelle inclinaison, par rapport à l'écran 20.

Sur les exemples représentés sur les figures 2A, 2B, 2E et 2F, on a représenté un capteur d'image dit auxiliaire 30aux, permettant de former une image auxiliaire I₃₀ₐᵤₓ du diffractogramme. De préférence, le capteur auxiliaire 30aux est disposé de façon symétrique du capteur d'image 30 par rapport à l'axe d'incidence Z. Le capteur d'image auxiliaire est optiquement couplé à la deuxième face 20₂ de l'écran par une optique de focalisation auxiliaire 25aux. La combinaison de l'image I₃₀ acquise par le capteur 30 et de l'image auxiliaire acquise I₃₀ₐᵤₓ par le capteur d'image auxiliaire 30aux permet d'obtenir une image dite résultante, sans déformation du diffractogramme. L'image résultante permet d'obtenir un diffractogramme présentant une symétrie de rotation.

Selon une variante, le capteur d'image 30 est mobile et peut occuper plusieurs positions autour du faisceau incident 12, une image étant acquise à chaque position. La combinaison des images ainsi formées permet d'obtenir une image résultante, sur laquelle le diffractogramme apparaît de façon peu ou pas déformée..

Selon une variante, l'écran 20 comporte un composant optique structuré, définissant par exemple une lentille de Fresnel. Une lentille de Fresnel comporte des structures annulaires concentriques agencées pour focaliser une image de grand diamètre selon une distance focale courte. La société DNP commercialise des écrans destinés à des applications en rétrodiffusion, basés sur des lentilles optiques structurées dans une face ou dans les deux faces d'un écran. Ces écrans sont désignés par le terme "optical rear projection screens". De tels écrans permettent d'augmenter la quantité de signal collectée par le capteur d'image.

Selon une variante, l'écran 20 comporte plusieurs guides de lumières s'étendant entre la première face 20₁ et la deuxième face 20₂, pour transporter le diffractogramme de la première face 20₁ vers la deuxième face 20₂. Il peut s'agir d'un panneau à fibres optiques, comportant un réseau de fibres optiques s'étendant les unes à côté des autres, entre la première face 20₁ et la deuxième face 20₂. La dimension, selon le plan XY, d'un tel écran peut atteindre plusieurs centaines de cm², par exemple 32,5 cm x 32,5 cm. Le diamètre de chaque fibre optique est compris entre 5 µm et 25 µm, l'ouverture numérique étant comprise entre 0.92 et 1. De tels panneaux sont par exemple commercialisés par la société Schott.

La figure 2C représente un écran formé de deux couches : une couche inférieure 21, définissant la première face 20₁ de l'écran, et une couche supérieure 22 définissant la deuxième face de l'écran 20₂. La couche inférieure 21 peut être diffusante, en étant par exemple constituée d'une plaque dépolie en verre ou en plastique, la surface dépolie correspondant à la première face 20₁. La couche supérieure 22 peut former une lentille de Fresnel ou une plaque transparente en verre, faisant office de couche de protection. Il n'est pas nécessaire que la couche supérieure présente une ouverture 23, du fait qu'elle est transparente.

La figure 2D représente un détail de l'ouverture 23, ainsi que le rayonnement incident 12 et le rayonnement rétrodiffusé 14. Le rayonnement incident 12 se propage jusqu'à l'objet 3, selon un axe Z, dit axe d'incidence. L'axe d'incidence Z est sensiblement perpendiculaire à la surface 3s de l'objet 3 à observer, ou sensiblement perpendiculaire à un plan XY, dit plan de l'échantillon, selon lequel s'étend le milieu de culture 4 de l'échantillon 2. Par sensiblement perpendiculaire, on entend perpendiculaire à une tolérance angulaire près, cette dernière étant de préférence inférieure à ±30°, ou de préférence inférieure à ±20°. Ainsi, le faisceau lumineux incident 12 atteint l'objet 3 selon un angle d'incidence sensiblement égal à 90°, à la tolérance angulaire près. On a également représenté le rayonnement rétrodiffusé 14 émanant de l'objet 3, et prenant la forme d'un cône 15 d'angle au sommet Ω. Il comporte une première composante, notée 14₁, et désignée par le terme "composante de réflexion", correspondant essentiellement à une réflexion spéculaire du faisceau incident 12 à la surface de l'échantillon, à laquelle s'ajoute l'ordre 0 de la diffraction. Il comporte une deuxième composante 14₂, s'étendant autour de la première composante, la deuxième composante 14₂ comportant l'information exploitable pour caractériser l'objet 3.

L'ouverture 23 ménagée dans l'écran est dimensionnée en fonction du diamètre du faisceau incident 12 émis par la source de lumière 10. Une partie du rayonnement rétrodiffusé 14 ne se propage pas jusqu'à l'écran 20, et n'apparaît donc pas sur le diffractogramme, du fait de la présence de l'ouverture 23. Cela bloque une transmission de la première composante 14₁ du rayonnement rétrodiffusé vers l'écran. Or, comme précédemment indiqué, la première composante 14₁ représente essentiellement une réflexion spéculaire du faisceau incident 12 sur l'objet 3 ; elle ne comporte pas, ou peu, d'information utile pour caractériser l'objet 3 observé. De plus, cette première composante est généralement intense. Sa non-transmission vers l'écran 20 permet de supprimer une contribution intense et peu informative sur le diffractogramme. Cela améliore la dynamique du diffractogramme. Le masquage, par l'ouverture 23, de la composante de réflexion 14₁ apparaît, sur les diffractogrammes, sous la forme d'une ombre. Cette ombre est désignée par une flèche noire sur le diffractogramme représenté sur la figure 2B.

La distance d entre l'échantillon 2 et l'écran 20 est avantageusement variable, comme illustré sur les figures 2E et 2F. En effet, comme précédemment indiqué, la distribution spatiale du rayonnement de rétrodiffusion 14 peut varier, ce dernier pouvant prendre la forme d'un cône 15 plus ou moins ouvert, et s'étendant, à partir de l'objet de façon divergente ou convergente. De ce fait, le support 6 de l'échantillon peut être monté sur une platine de translation autorisant une translation parallèlement à l'axe d'incidence Z. Les figures 2E et 2F représentent un échantillon 2 situé respectivement à une première distance d = d₁ et à une deuxième distance d = d₂ de l'écran 20, avec d₁ > d₂. Le déplacement du support 6 peut être commandé par le processeur 40. La plage de variation de la distance est typiquement de 3 cm à 20 cm, voire 30 cm. La distance est déterminée en fonction du diffractogramme formé sur l'écran 20, de façon que le diffractogramme s'étende selon une surface la plus grande possible, tout en restant compatible avec le champ d'observation du capteur d'image 30, ce dernier dépendant de la dimension du capteur d'image et du système optique de focalisation 25.

Le réglage de la distance peut se faire manuellement, ou en mettant en oeuvre un algorithme basé sur une reconnaissance du contour délimitant le diffractogramme. Un tel algorithme peut par exemple utiliser un filtrage de Canny. Lorsque ce contour a été détecté, la distance est ajustée de façon que l'aire du diffractogramme, sur l'écran 20, dépasse une valeur seuil prédéterminée. L'ajustement de la distance d permet de prendre en compte la variabilité du rayonnement de rétrodiffusion en fonction des différents types d'objets à caractériser. Selon un mode de réalisation, lorsqu'une distance optimale a été déterminée, permettant de maximiser l'aire du diffractogramme projeté sur l'écran, une image du diffractogramme est acquise. La distance est ensuite augmentée, de façon à vérifier l'absence de rayonnement rétrodiffusé à l'extérieur du diffractogramme, correspondant à la distance optimale, préalablement observé. De préférence, le support 6 est également mobile dans le plan de l'échantillon XY. Cela permet d'effectuer un centrage du faisceau lumineux incident 12 sur l'objet 3. Cela permet d'effectuer une analyse quelle que soit la position de l'objet 3 dans l'échantillon 2. Un tel centrage peut être ajusté sur un critère de symétrie du diffractogramme. En effet, lorsque le faisceau incident est centré sur l'objet, le diffractogramme présent sur l'écran présente une symétrie de révolution. La symétrie peut par exemple être quantifiée par la forme du contour du diffractogramme.

Quel que soit l'exemple, un système optique de mise en forme 11 peut être associé à la source de lumière 10, de façon à former un faisceau incident 12 collimaté, selon des principes connus de l'homme du métier. La figure 2G représente un exemple de système optique de mise en forme. Il comporte une succession de composants optiques usuels : une lentille achromat 110, un sténopé 111 de 50 µm, une lentille convergente 112 et un expanseur de faisceau 113 . Le système optique de mise en forme 11 peut comprendre, de façon optionnelle, un convertisseur de faisceau de type "flat top" 114 suivi d'un réducteur de faisceau 115. L'expanseur de faisceau 113 permet d'ajuster la taille du faisceau laser, de façon à que cette dernière se rapproche de la taille de l'objet à observer. L'expanseur 113 peut être constitué d'un ensemble de deux lentilles à focale variable, programmable par le processeur 40. Le convertisseur de faisceau 114 permet d'ajuster la distribution de l'intensité dans le faisceau.

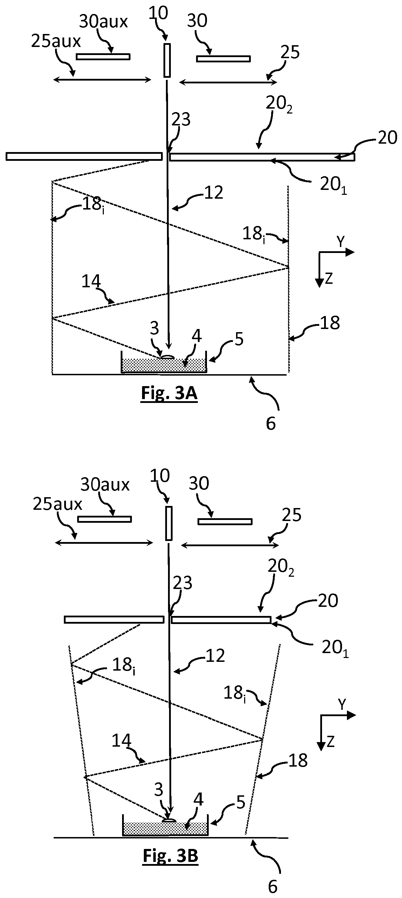

La distribution spatiale du rayonnement rétrodiffusé 14 peut varier significativement selon les objets observés. Dans certains cas, elle s'étend selon une plage angulaire très élevée de part et d'autre de l'axe d'incidence Z. C'est notamment le cas lorsque l'objet, en l'occurrence une colonie bactérienne, présente une morphologie bombée, une telle morphologie étant par exemple observée sur des colonies bactériennes de staphylocoques. Dans un tel cas de figure, la taille de l'écran 20 doit être importante pour obtenir un diffractogramme complet, et en particulier prenant en compte les grands angles de rétrodiffusion, typiquement supérieurs à 65°. Le terme angle de rétrodiffusion désigne l'angle entre un rayonnement rétrodiffusé 14 émanant de l'objet et l'axe d'incidence Z. Il est également possible d'ajuster la distance entre l'écran 20 et l'objet 3, comme précédemment indiqué. Cela permet notamment d'obtenir un diffractogramme dont le diamètre correspond à un gabarit prédéterminé, par exemple un diamètre compris entre 15 et 20 cm. La figure 3A décrit une variante permettant de conserver une taille d'écran 20 raisonnable tout en permettant une prise en compte du rayonnement de rétrodiffusion 14 émanant de l'objet sous des grands angles de rétrodiffusion. Selon cette variante, un réflecteur annulaire 18, s'étendant parallèlement à l'axe d'incidence Z, est disposé entre l'objet 3 et l'écran 20, autour de tout ou partie de l'objet 3. Le réflecteur annulaire 18 permet de réfléchir une partie du rayonnement rétrodiffusé 14 vers l'écran 20. Il peut s'agir d'un réflecteur tubulaire coaxial de l'axe d'incidence Z. La figure 3A représente un réflecteur annulaire cylindrique. Sa hauteur et son diamètre peuvent être respectivement de 6 cm et 17.5 cm. Il peut s'agir d'un cylindre dont la paroi interne 18i est réfléchissante. Par exemple, la paroi interne 18i a fait l'objet d'un dépôt d'une fine couche métallique, par exemple de l'aluminium. Le réflecteur annulaire 18 peut également être de forme conique, comme représenté sur la figure 3B. Un tel réflecteur conique peut avoir un petit diamètre égal à 19 cm, un grand diamètre égal à 20 cm, et une hauteur de 3 cm. L'angle d'inclinaison de la paroi interne, par rapport à l'axe Z, est par exemple de 13°. L'angle de la paroi interne 18i peut être dimensionné de telle sorte que le rayonnement rétrodiffusé 14 présentant l'angle de rétrodiffusion le plus élevé ne subisse qu'une seule réflexion avant d'atteindre l'écran 20. De préférence, au moins un diamètre du réflecteur annulaire 18 est supérieur à 2 fois le diamètre de l'enceinte 5.

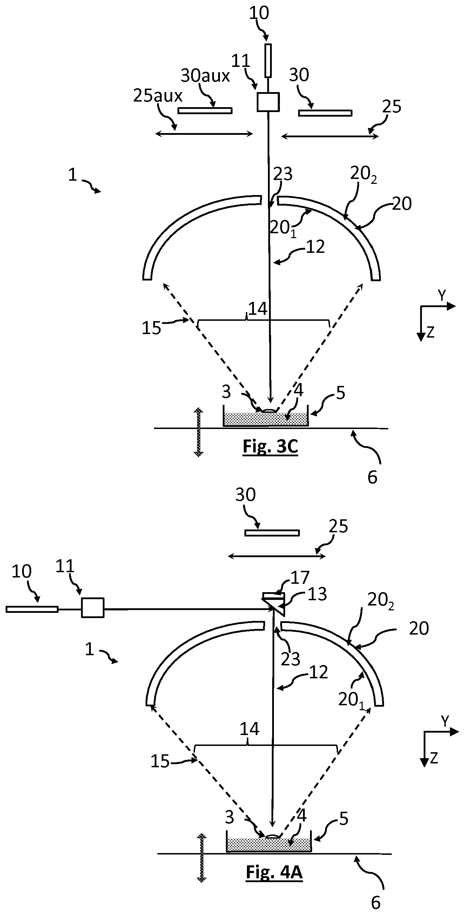

La figure 3C représente une variante pouvant être utilisée en complément ou en alternative au réflecteur annulaire décrit en lien avec les figures 3A ou 3B. Selon cette variante, l'écran 20 n'est pas plan et prend une forme courbée s'incurvant vers l'échantillon 2. Cela facilite également une collecte, par l'écran, de rayonnements rétrodiffusés selon des angles de rétrodiffusion importants. La courbure de l'écran 20 peut être régulière ou non. L'écran peut par exemple avoir la forme d'un dôme. L'écran peut également décrire une courbure en présentant des facettes planes.

Par s'incurvant vers l'échantillon, on entend que l'écran décrit une courbure dont le centre est situé entre l'échantillon et l'écran, ou plus généralement dans un demi-espace délimité par l'écran et comprenant l'échantillon. Ainsi, l'écran a une forme concave, de façon à définir un espace s'étendant entre l'écran et l'échantillon, l'espace étant tel que, quels que soient deux points dudit espace, le segment reliant lesdits points est inclus dans l'espace.

La figure 4A montre un mode de réalisation dans lequel l'écran 20 est courbé, comme décrit en lien avec la figure 3C. Le dispositif comporte un élément réfléchissant 13, recevant le faisceau incident émis par la source de lumière 10 et le réfléchissant vers l'objet 3, selon l'axe d'incidence Z. Dans cet exemple, le faisceau incident 12 est émis selon une direction parallèle à la surface du milieu de culture 4, puis est réfléchi vers l'échantillon selon une incidence normale à ce dernier. Un avantage de ce mode de réalisation est que le capteur d'image 30 et le système optique 25 peuvent être centrés par rapport à l'axe d'incidence Z, ce qui revient à un centrage par rapport à l'axe de rétrodiffusion -Z. Cela permet d'acquérir une image symétrique du diffractogramme formé sur l'écran 20, avec un seul capteur d'image. L'image acquise peut alors conserver alors une invariance par rotation. Lorsque l'écran 20 est courbé, le système optique 25 est de préférence un objectif grand angle, permettant d'obtenir une grande profondeur de champ de façon à former une image nette du diffractogramme apparaissant sur la deuxième surface de l'écran 20₂. L'ouverture 23 est de préférence située au niveau de l'apex de l'écran 20.

La surface de l'élément réfléchissant 13 est la plus faible possible, de façon à ne pas interférer avec le rayonnement rétrodiffusé 14 émanant de l'objet 3. Elle est de préférence inférieure à 5 cm², et encore de préférence inférieure à 2 cm², voire inférieure à 1 cm². La surface de l'élément réfléchissant 13 est de préférence adaptée au diamètre du faisceau émis par la source de lumière 10. L'élément réféchissant 13 peut être fixé sur un support 17. De préférence l'élément réfléchissant 13 et/ou le support 17 sur lequel il est fixé absorbent au moins 30%, et avantageusement au moins 50%, voire 80% ou 90% du rayonnement rétrodiffusé 14 émis par l'objet. Cela permet d'éviter une transmission de la première composante 14₁ du rayonnement de rétrodiffusion, précédemment décrite, du rayonnement rétrodiffusé 14, vers le capteur d'image 30.

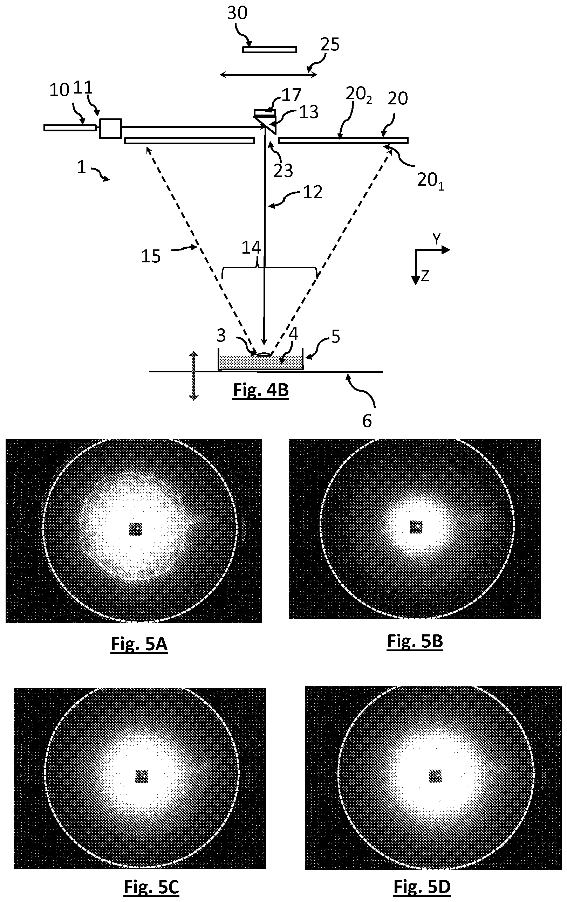

La figure 4B représente une variante de ce mode de réalisation, selon laquelle l'écran 20 est plat.

L'image obtenue sur le capteur d'image 20 peut permettre de caractériser l'objet 3. La caractérisation peut être une identification. Pour cela, des caractéristiques de l'image sont déterminées, et comparées à des caractéristiques de calibration établies sur des objets étalons. Elles peuvent également faire l'objet d'une classification sur la base desdites caractéristiques de calibration. La demande de brevet WO2014184390 décrit un procédé de classification de colonies bactériennes basé sur une projection de l'image sur une base de polynômes de Zernike orthogonaux. D'autres algorithmes de classification, par exemple une analyse en composantes principales, sont envisageables. Une telle classification a pour objet de réduire l'information spatiale de l'image en un jeu de coordonnées, sur la base duquel l'identification du microorganisme est obtenue.

### Essais expérimentaux

Des essais expérimentaux ont été réalisés, en mettant en oeuvre le mode de réalisation représenté sur les figures 4A. Les principaux composants utilisés ont été les suivants :
- source de lumière 10 : source laser référence LCGFP-D-532-10C-F - fournie par Laser components.
- système optique de mise en forme 11 : lentille achromat Thorlabs AC254-030-A-ML - A280TM-A ; sténopé Thorlabs - P50S, lentille convergente Thorlabs A280TM-A.
- Enceinte de l'échantillon : boîte de Pétri de diamètre 90 mm - Biomérieux.
- Système optique de focalisation : LM5JC10M - Kowa.
- Caméra : UI-1492ME - IDS ou AVGT3300 - Allied Vision.
- Element réfléchissant : miroir incliné à 45°.

L'ensemble est placé dans l'obscurité.

Au cours de ces essais, on a observé différents types de colonies bactériennes. Lors de chaque opération, le centrage du faisceau laser incident 12 sur la colonie est réalisée visuellement, par l'opérateur.

Les figures 5A à 5D sont des exemples d'images de diffractogrammes obtenus en observant des colonies bactériennes se développant sur une gélose COS (Columbia Blood Ship) décrite en lien avec l'art antérieur. L'écran 20 utilisé est en forme de dôme, en verre dépoli. Il s'agit d'un écran translucide, non transparent. On a représenté, sur chacune de ces figures, la bordure circulaire de l'écran par un contour en pointillés blancs. Les colonies bactériennes observées sur chacune de ces figures sont les suivantes.
- figure 5A : *Staphylococcus maltophilia ;*
- figure 5B : *Staphylococcus saprophyticus ;*
- figure 5C : *Staphylococcus warneri;*
- figure 5D : *Pseudomonas putida ;*

Le diffractogramme de chaque colonie se projette entièrement sur l'écran 20, sans dépasser de ce dernier, ce qui permet d'en acquérir une image complète à l'aide du capteur d'image 30. La distance entre l'apex de l'écran et chaque colonie était de 4 cm. Le diamètre du faisceau laser était de 1 mm.

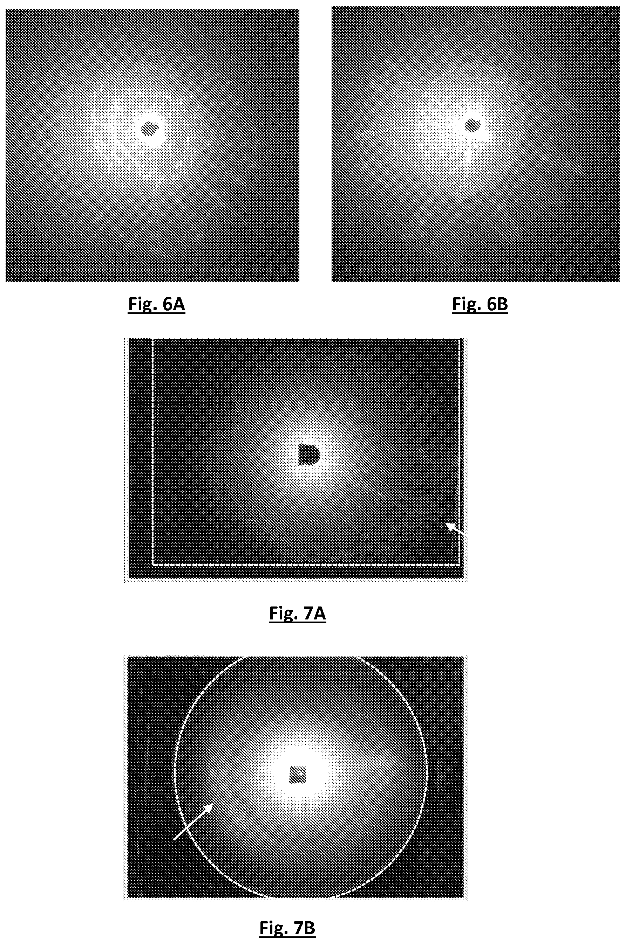

Les figures 6A et 6B ont été obtenues en mettant en oeuvre l'exemple décrit en lien avec la figure 2A, sans le capteur d'image auxiliaire 30 aux. L'écran 20 utilisé est une feuille plane de papier calque. Les principaux composants du dispositif sont ceux listés ci-dessus. Les colonies bactériennes observées, se développant sur un milieu de culture COS, sont :
- figure 6A : *Escherichia coli ;*
- figure 6B : *Pseudomonas putida.*

On obtient des images de diffractogrammes exploitables, mais présentant une dissymétrie du fait du décalage du capteur d'image 30 par rapport à l'axe de rétrodiffusion (-Z). La durée d'acquisition de chaque image était de 136 ms. Les distances entre la colonie et l'écran étaient respectivement de 10 cm (fig. 6A) et 7.5 cm (fig. 6B).

L'essai représenté en lien avec les figures 7A et 7B permet de comparer un mode de réalisation mettant en oeuvre un écran 20 plan, tel que représenté sur la figure 4B, avec un mode de réalisation mettant en oeuvre un écran 20 en forme de dôme, selon la figure 4A. Lors de chaque essai, on a utilisé les composants du dispositif listés en lien avec les figures 5A à 5D.Sur chacune de ces figures, on distingue l'ombre formée par l'élément réfléchissant 13 et de son support 17, au centre du diffractogramme. La colonie bactérienne est de type *Stenotrophomonas maltophilia* sur un milieu de culture COS. Dans le dispositif mettant en oeuvre un écran plan, tel que représenté sur la figure 4B, l'écran 20 était est une toile rétro gris fournie par Multivision. La distance entre l'écran 20 et la colonie est faible, inférieure à 1cm, la colonie étant en quasicontact avec l'écran. On a obtenu le diffractogramme représenté sur la figure 7A. La bordure de l'écran 20 est repérée sur l'image par un cadre en pointillés blancs. La flèche blanche indique la périphérie du diffractogramme. Une telle colonie bactérienne produit un rayonnement de rétrodiffusion 14 se propageant selon des angles de rétrodiffusion élevés par rapport à l'axe du faisceau incident, ces angles dépassant 65°. Il en résulte un diffractogramme s'étendant jusqu'à la bordure de l'écran.

En mettant en oeuvre le mode de réalisation utilisant un écran 20 en forme de dôme, tel que représenté sur la figure 4A, le diffractogramme est plus compact, comme représenté sur la figure 7B. Dans cet essai, on a mis en oeuvre l'écran 20 précédemment décrit en lien avec les figures 5A à 5D. La forme de l'écran permet d'obtenir un diffractogramme complet, incluant les angles de rétrodiffusion élevés.

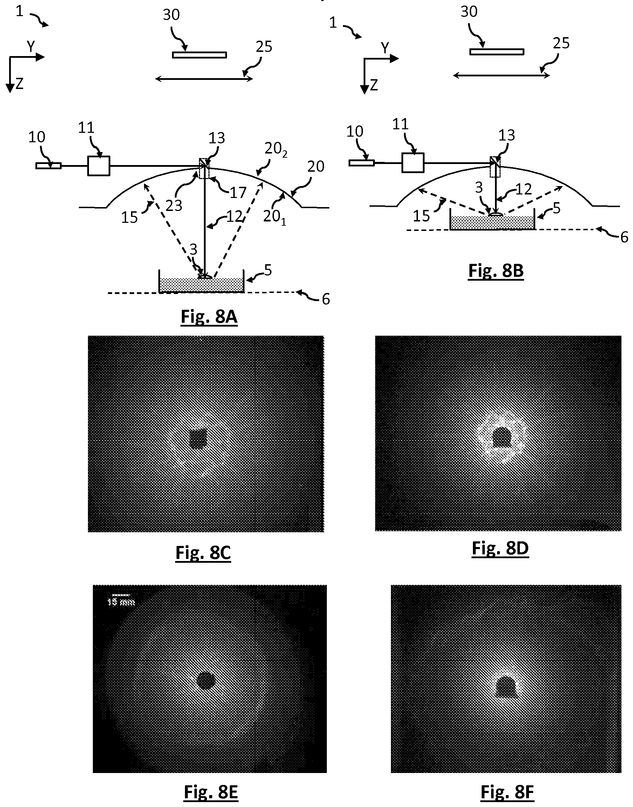

Au cours d'une autre série d'essais, on a comparé des diffractogrammes respectivement obtenus à l'aide d'un écran 20 décrivant une portion d'un dôme et d'un écran plat. Les figures 8A et 8B illustrent une configuration proche de celle décrite en lien avec la figure 4A. L'écran 20 utilisé est un écran fourni par Draper Inc, en acrylique, référence IRUS Screen, custom vaccuum formed ¼" acrylic, tint Cine25, coating HC. Une ouverture 23 de diamètre 15 mm est pratiquée au niveau de l'apex de l'écran. Dans l'ouverture, on a inséré un support cylindrique 17, maintenant un élément réfléchissant 13. Ce dernier permet de réfléchir le faisceau incident 12 vers l'objet analysé 3. Comme précédemment indiqué, l'objet est mobile par rapport à l'écran. Il est disposé sur un support 6 apte à se translater par rapport à l'écran 20, en fonction du cône 15 formé par le rayonnement rétrodiffusé par l'objet. Lorsque l'angle au sommet du cône 15 est faible, l'objet peut être éloigné de l'écran (figure 8A), de façon à ce que la surface du diffractogramme apparaissant sur l'image du capteur d'image 30 soit suffisamment étendue. Lorsqu'à l'inverse, l'angle au sommet du cône 15 est élevé (figure 8B), l'objet est rapproché de l'écran, de façon à ce que le rayonnement de rétrodiffusion, émis selon un angle de rétrodiffusion élevé, puisse être représenté sur le diffractogramme. Le diamètre du faisceau incident 12 est de 2 mm, la puissance d'éclairement étant de 4 mW.

Les figures 8C et 8D représentent des diffractogrammes obtenus à partir du capteur d'image 30 respectivement en utilisant une configuration telle que représentée :
- sur la figure 8A ;
- et sur la figure 4B, en mettant en oeuvre un écran plat réalisé selon le même matériau et la même teinte, même revêtement de surface (coating) que l'écran incurvé décrit en lien avec les figures 8A et 8B.

L'échantillon est une colonie de bactéries de type *Pseudomonas putida,* produisant un rayonnement de rétrodiffusion selon un cône 15 d'angle relativement faible. Le milieu de culture est COS, précédemment décrit. Le temps de pose est de 600 ms (figure 8C) et 900 ms (figure 8D). Les images sont de bonne qualité. On observe que l'atténuation de la composante de réflexion 14₁ permet de visualiser convenablement le diffractogramme, et en particulier sa périphérie, sans éblouissement. Cette atténuation se traduit par une ombre formée au centre des images. Ainsi, on évite de saturer les pixels du capteur d'image par un rayonnement intense, ce qui permet de mieux mettre en évidence les rayonnements moins intenses, par exemple au niveau de la périphérie du diffractogramme.

Les figures 8E et 8F représentent des diffractogrammes obtenus à partir du capteur d'image 30 respectivement en utilisant une configuration telle que représentée :
- sur la figure 8B ;
- et sur la figure 4B, en mettant en oeuvre un écran plat réalisé selon le même matériau et la même teinte, même revêtement de surface (coating) que l'écran incurvé décrit en lien avec les figures 8A et 8B.

L'échantillon est une colonie de bactéries de type *Stenotrophomonas maltophilia,* produisant un rayonnement de rétrodiffusion selon un angle relativement élevé. Le milieu de culture est COS, précédemment décrit. On observe que l'atténuation de la composante de réflexion 14₁ permet de visualiser convenablement le diffractogramme, et en particulier sa périphérie, sans éblouissement. De plus, en comparant ces deux figures, la forme incurvée de l'écran 20 (cf. figure 8E) permet de collecter davantage de rayonnement rétrodiffusé émis sous des angles élevés par rapport à l'angle d'incidence du faisceau 12. Le diffractogramme obtenu est plus complet.

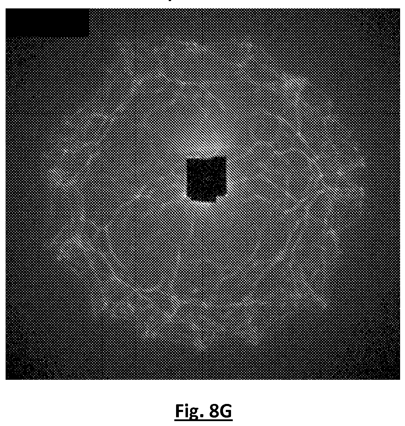

La figure 8G représente un diffractogramme de deux colonies de *Yerinia ruckerii,* acquis avec un dispositif selon la figure 8B. Cette figure permet d'apprécier la qualité de l'image obtenue à l'aide de l'écran incurvé.

Le procédé n'étant pas destructif, plusieurs images d'une même colonie bactérienne, à différents stades d'incubation, peuvent être réalisées, de façon à apprécier la capacité de la colonie à se développer, ou à résister à un agent antibiotique ou antibactérien. Dans ce cas, la caractérisation de l'objet représente l'aptitude de ce dernier à se développer.

Le procédé peut également permettre un dénombrement d'objets présents à la surface d'un échantillon.

L'invention pourra être mise en oeuvre en support à différents types d'examens, de type test de stérilité, test de susceptibilité à des antibiotiques, test de susceptibilités à des antibactériens ou aux bactériophages, criblage de substances antibactériennes, identification, dénombrement. L'invention peut également s'appliquer à l'observation et à la caractérisation d'autres types de microorganismes, comme des levures, des champignons ou des microalgues.

## Revendications

1. Dispositif pour observer un objet (3), présent dans un échantillon (2) comportant :
- un support (6), apte à recevoir l'échantillon (2),
- une source de lumière (10), apte à émettre un faisceau lumineux (12), dit faisceau lumineux incident, pour illuminer l'objet ;
- un capteur d'image (30), configuré pour acquérir une image représentative d'un rayonnement (14) rétrodiffusé par l'objet sous l'effet d'une illumination par le faisceau lumineux incident (12) ;
- un écran (20), s'étendant en regard du support (6), de manière à être exposé à un rayonnement (14) rétrodiffusé par l'objet lorsque ce dernier est illuminé par le faisceau lumineux incident (12), de façon à former, sur l'écran, une image (I₂₀), dite diffractogramme, représentative dudit rayonnement rétrodiffusé (14);
- l'écran (20) comportant une première face (20₁) exposée au rayonnement rétrodiffusé (14);
- l'écran s'étendant entre la source de lumière (10) et l'objet (3), et comportant une ouverture (23), à travers laquelle se propage le faisceau lumineux incident (12) avant d'atteindre l'objet (3), l'écran comportant une deuxième face (20₂), de façon que le diffractogramme formé sur la première face (20₁) apparaisse sur la deuxième face ;
- le capteur d'image (30) étant configuré pour acquérir une image (I₃₀) du diffractogramme (I₂₀) formé sur l'écran, l'écran étant disposé entre le capteur d'image (30) et l'objet (3), le capteur d'image (30) étant couplé à la deuxième face de l'écran (202) par une optique de focalisation (25) ;
le dispositif étant **caractérisé en ce qu'**il comporte un élément réfléchissant (13), apte à diriger le faisceau lumineux émis par la source de lumière vers l'objet (3), à travers l'ouverture (23), l'élément réfléchissant étant disposé entre l'objet (3) et le capteur d'image (30), et **en ce que** :
- l'élément réfléchissant (13) absorbe au moins 30% du rayonnement rétrodiffusé (14) émis par l'objet ;
- et/ou l'élément réfléchissant (13) est fixé sur un support (17), le support absorbant au moins 30% du rayonnement rétrodiffusé (14) émis par l'objet.

2. Dispositif selon la revendication 1, dans lequel le faisceau incident (12) atteint l'objet (3) selon une incidente perpendiculaire ou sensiblement perpendiculaire à ce dernier, le terme sensiblement perpendiculaire désignant une incidence perpendiculaire avec une tolérance angulaire de ± 30°.

3. Dispositif selon l'une quelconque des revendications précédentes, dans lequel le capteur d'image (30) est centré par rapport à l'axe d'incidence selon lequel se propage le faisceau indicent (12) atteignant l'objet (3).

4. Dispositif selon l'une quelconque des revendications précédentes, dans lequel le diamètre de l'ouverture (23) est inférieur à 2 cm.

5. Dispositif selon l'une quelconque des revendications précédentes, dans lequel l'écran (20) a une forme incurvée et/ou dans lequel l'écran (20) est translucide.

6. Dispositif selon l'une quelconque des revendications précédentes, dans lequel l'écran (20) comporte un guide de lumière pour transporter une lumière entre la première face (20₁) et la deuxième face (20₂), et/ou dans lequel l'écran comporte plusieurs fibres optiques s'étendant entre la première face (20₁) et la deuxième face (20₂).

7. Dispositif selon l'une quelconque des revendications précédentes, dans lequel le support (6) est mobile par rapport à l'écran (20), la distance (d) entre le support et l'écran pouvant être ajustée.

8. Dispositif selon l'une quelconque des revendications précédentes, dans lequel le faisceau incident (12) se propage entre l'écran (20) et l'objet (3) autour d'un axe dit axe d'incidence (Z), le dispositif comportant un réflecteur dit annulaire (18), s'étendant autour de l'axe d'incidence, entre l'échantillon (2) et l'écran (20), le réflecteur annulaire étant apte à réfléchir une partie du rayonnement rétrodiffusé (14) vers l'écran (20).

9. Procédé d'observation d'un objet (3) présent dans un échantillon (2), l'échantillon s'étendant face à un écran (20) comportant une première face (20₁), le procédé comportant les étapes suivantes :
a) illumination de l'objet (3) à l'aide d'un faisceau lumineux incident (12) émis par une source de lumière (10), l'écran s'étendant entre la source de lumière et l'objet (3), le faisceau lumineux incident (12) se propageant jusqu'à l'objet à travers une ouverture (23) ménagée dans l'écran ;
b) exposition d'une première face (20₁) de l'écran à un rayonnement lumineux (14) rétrodiffusé par l'échantillon, de façon à former, sur la première face (20₁), une image, dite diffractogramme, représentative dudit rayonnement rétrodiffusé (14), l'écran comportant une deuxième face (20₂), de façon que le diffractogramme formé sur la première face (20₁) apparaisse sur la deuxième face (20₂);
c) acquisition d'une image du diffractogramme formé sur l'écran par un capteur d'image (30), l'écran s'étendant entre le capteur d'image (30) et l'objet (3), le capteur d'image (30) étant couplé à la deuxième face (20₂) par une optique de focalisation (25);
le procédé étant **caractérisé en ce que** :
- le faisceau lumineux émis par la source de lumière est dirigé vers l'objet (3), à travers l'ouverture (23), par un élément réfléchissant (13) ;
- l'élément réfléchissant (13) est disposé entre l'objet (3) et le capteur d'image (30). ;
- l'élément réfléchissant (13) absorbe au moins 30% du rayonnement rétrodiffusé (14) émis par l'objet ;
- et/ou l'élément réfléchissant (13) est fixé sur un support (17), le support absorbant au moins 30% du rayonnement rétrodiffusé (14) émis par l'objet.

10. Procédé selon la revendication 9, comportant, suite à l'étape c), une étape d'ajustement de la distance (d) entre l'échantillon (2) et l'écran (20) en fonction de l'image acquise par le capteur d'image (30), les étapes a) à c) étant répétées après l'ajustement de ladite distance.

11. Procédé selon l'une quelconque des revendications 9 ou 10, comportant une étape d) de caractérisation de l'objet (3) à partir de l'image acquise par le capteur d'image.

12. Procédé selon la revendication 11, dans lequel l'étape d) comporte :
- une détermination de caractéristiques de l'image ;
- une identification de l'objet à l'aide desdites caractéristiques et de caractéristiques de calibration établies en mettant en oeuvre les étapes a) à c) du procédé à l'aide d'un échantillon étalon.

13. Procédé selon l'une quelconque des revendications 9 à 12, dans lequel l'objet comporte un microorganisme.

## Patentansprüche

1. Vorrichtung zum Beobachten eines Objekts (3), das in einer Probe (2) vorhanden ist, umfassend:
- einen Träger (6), der geeignet ist, die Probe (2) aufzunehmen,
- eine Lichtquelle (10), die geeignet ist, einen Lichtstrahl (12), einfallender Lichtstrahl genannt, auszusenden, um das Objekt zu beleuchten;
- einen Bildsensor (30), der dazu ausgestaltet ist, ein Bild zu erfassen, das für eine Strahlung (14) repräsentativ ist, die von dem Objekt unter der Wirkung einer Beleuchtung durch den einfallenden Lichtstrahl (12) rückgestreut wird;
- einen Schirm (20), der sich gegenüber dem Träger (6) erstreckt, so dass er einer Strahlung (14) ausgesetzt ist, die von dem Objekt rückgestreut wird, wenn Letzteres von dem einfallenden Lichtstrahl (12) beleuchtet wird, so dass auf dem Schirm ein Bild (I₂₀), Diffraktogramm genannt, gebildet wird, das für die rückgestreute Strahlung (14) repräsentativ ist;
- wobei der Schirm (20) eine erste Seite (20₁) umfasst, die der rückgestreuten Strahlung (14) ausgesetzt ist;
- wobei sich der Schirm zwischen der Lichtquelle (10) und dem Objekt (3) erstreckt und eine Öffnung (23) umfasst, durch die hindurch sich der einfallende Lichtstrahl (12) ausbreitet, bevor er das Objekt (3) erreicht, wobei der Schirm eine zweite Seite (20₂) umfasst, so dass das auf der ersten Seite (20₁) gebildete Diffraktogramm auf der zweiten Seite erscheint;
- wobei der Bildsensor (30) dazu ausgestaltet ist, ein Bild (I₃₀) des auf dem Schirm gebildeten Diffraktogramms (I₂₀) zu erfassen, wobei der Schirm zwischen dem Bildsensor (30) und dem Objekt (3) angeordnet ist, wobei der Bildsensor (30) mit der zweiten Seite des Schirms (20₂) durch eine Fokussieroptik (25) gekoppelt ist;
wobei die Vorrichtung **dadurch gekennzeichnet ist, dass** sie ein reflektierendes Element (13) umfasst, das geeignet ist, den von der Lichtquelle ausgesendeten Lichtstrahl zu dem Objekt (3) durch die Öffnung (23) hindurch zu lenken, wobei das reflektierende Element zwischen dem Objekt (3) und dem Bildsensor (30) angeordnet ist, und dadurch, dass:
- das reflektierende Element (13) mindestens 30 % der von dem Objekt ausgesendeten rückgestreuten Strahlung (14) absorbiert;
- und/oder das reflektierende Element (13) an einem Träger (17) befestigt ist, wobei der Träger mindestens 30 % der von dem Objekt ausgesendeten rückgestreuten Strahlung (14) absorbiert.

2. Vorrichtung nach Anspruch 1, bei welcher der einfallende Strahl (12) das Objekt (3) entlang eines zu Letzterem senkrechten oder im Wesentlichen senkrechten Einfallslots erreicht, wobei der Ausdruck "im Wesentlichen senkrecht" einen senkrechten Einfall mit einer Winkeltoleranz von ± 30° bezeichnet.

3. Vorrichtung nach einem der vorhergehenden Ansprüche, bei welcher der Bildsensor (30) in Bezug auf die Einfallsachse, entlang der sich der das Objekt (3) erreichende einfallende Strahl (12) ausbreitet, zentriert ist.

4. Vorrichtung nach einem der vorhergehenden Ansprüche, bei welcher der Durchmesser der Öffnung (23) weniger als 2 cm beträgt.

5. Vorrichtung nach einem der vorhergehenden Ansprüche, bei welcher der Schirm (20) eine gebogene Form hat und/oder bei welcher der Schirm (20) transluzent ist.

6. Vorrichtung nach einem der vorhergehenden Ansprüche, bei welcher der Schirm (20) einen Lichtleiter umfasst, um ein Licht zwischen der ersten Seite (20₁) und der zweiten Seite (20₂) zu transportieren, und/oder bei welcher der Schirm mehrere optische Fasern umfasst, die sich zwischen der ersten Seite (20₁) und der zweiten Seite (20₂) erstrecken.

7. Vorrichtung nach einem der vorhergehenden Ansprüche, bei welcher der Träger (6) in Bezug auf den Schirm (20) beweglich ist, wobei der Abstand (d) zwischen dem Träger und dem Schirm angepasst werden kann.

8. Vorrichtung nach einem der vorhergehenden Ansprüche, bei welcher sich der einfallende Strahl (12) zwischen dem Schirm (20) und dem Objekt (3) um eine Achse herum, Einfallsachse (Z) genannt, ausbreitet, wobei die Vorrichtung einen Reflektor, Ringreflektor (18) genannt, umfasst, der sich um die Einfallsachse herum zwischen der Probe (2) und dem Schirm (20) erstreckt, wobei der Ringreflektor geeignet ist, einen Teil der rückgestreuten Strahlung (14) zu dem Schirm (20) hin zu reflektieren.

9. Verfahren zum Beobachten eines Objekts (3), das in einer Probe (2) vorhanden ist, wobei sich die Probe gegenüber einem Schirm (20) erstreckt, der eine erste Seite (20₁) umfasst, wobei das Verfahren die folgenden Schritte umfasst:
a) Beleuchten des Objekts (3) mithilfe eines einfallenden Lichtstrahls (12), der von einer Lichtquelle (10) ausgesendet wird, wobei sich der Schirm zwischen der Lichtquelle und dem Objekt (3) erstreckt, wobei sich der einfallende Lichtstrahl (12) bis zu dem Objekt durch eine in dem Schirm ausgebildete Öffnung (23) hindurch ausbreitet;
b) Aussetzen einer ersten Seite (20₁) des Schirms gegenüber einer Lichtstrahlung (14), die von der Probe rückgestreut wird, so dass auf der ersten Seite (20₁) ein Bild, Diffraktogramm genannt, gebildet wird, das für die rückgestreute Strahlung (14) repräsentativ ist, wobei der Schirm eine zweite Seite (20₂) umfasst, so dass das auf der ersten Seite (20₁) gebildete Diffraktogramm auf der zweiten Seite (20₂) erscheint;
c) Erfassen eines Bildes des auf dem Schirm gebildeten Diffraktogramms durch einen Bildsensor (30), wobei sich der Schirm zwischen dem Bildsensor (30) und dem Objekt (3) erstreckt, wobei der Bildsensor (30) mit der zweiten Seite (20₂) durch eine Fokussieroptik (25) gekoppelt ist;
wobei das Verfahren **dadurch gekennzeichnet ist, dass**:
- der von der Lichtquelle ausgesendete Lichtstrahl zu dem Objekt (3) hin durch die Öffnung (23) hindurch durch ein reflektierendes Element (13) gelenkt wird;
- das reflektierende Element (13) zwischen dem Objekt (3) und dem Bildsensor (30) angeordnet ist;
- das reflektierende Element (13) mindestens 30 % der von dem Objekt ausgesendeten rückgestreuten Strahlung (14) absorbiert;
- und/oder das reflektierende Element (13) an einem Träger (17) befestigt ist, wobei der Träger mindestens 30 % der von dem Objekt ausgesendeten rückgestreuten Strahlung (14) absorbiert.

10. Verfahren nach Anspruch 9, umfassend, nach dem Schritt c), einen Schritt des Anpassens des Abstands (d) zwischen der Probe (2) und dem Schirm (20) in Abhängigkeit von dem vom Bildsensor (30) erfassten Bild, wobei die Schritte a) bis c) nach dem Anpassen des Abstands wiederholt werden.

11. Verfahren nach einem der Ansprüche 9 oder 10, umfassend einen Schritt d) des Charakterisierens des Objekts (3) anhand des von dem Bildsensor erfassten Bildes.

12. Verfahren nach Anspruch 11, bei dem der Schritt d) umfasst:
- ein Bestimmen von Merkmalen des Bildes;
- ein Identifizieren des Objekts mithilfe der Merkmale und von Kalibrierungsmerkmalen, die ermittelt werden, indem die Schritte a) bis c) des Verfahrens mithilfe einer Standardprobe durchgeführt werden.

13. Verfahren nach einem der Ansprüche 9 bis 12, bei dem das Objekt einen Mikroorganismus umfasst.

## Claims

1. Device for observing an object (3), present in a sample (2), comprising:
- a holder (6), able to receive the sample (2);
- a light source (10), able to emit a light beam (12), called the incident light beam, in order to illuminate the object;
- an image sensor (30), configured to acquire an image representative of radiation (14) back-scattered by the object under the effect of an illumination by the incident light beam (12);
- a screen (20), lying facing the holder (6), so as to be exposed to radiation (14) back-scattered by the object when the latter is illuminated by the incident light beam (12), so as to form, on the screen, an image (I₂₀), called a scattergram, representative of said back-scattered radiation (14);
- the screen (20) comprising a first face (20₁) exposed to the back-scattered radiation (14);
- the screen lying between the light source (10) and the object (3), and comprising an aperture (23), through which the incident light beam (12) propagates before reaching the object (3), the screen comprising a second face (20₂), so that the scattergram formed on the first face (20₁) appears on the second face;
- the image sensor (30) being configured to acquire an image (I₃₀) of the scattergram (I₂₀) formed on the screen, the screen being placed between the image sensor (30) and the object (3), the image sensor (30) being coupled to the second face of the screen (202) by a focusing optic (25);
the device being **characterized in that** it comprises a reflective element (13), able to direct the light beam emitted by the light source toward the object (3), through the aperture (23), the reflective element being placed between the object (3) and the image sensor (30), and **in that**:
- the reflective element (13) absorbs at least 30% of the back-scattered radiation (14) emitted by the object;
- and/or the reflective element (13) is fastened to a holder (17), the holder absorbing at least 30% of the back-scattered radiation (14) emitted by the object.

2. Device according to Claim 1, wherein the incident beam (12) reaches the object (3) at an incidence perpendicular or substantially perpendicular to the latter, the term substantially perpendicular designating a perpendicular incidence with an angular tolerance of ± 30°.

3. Device according to either one of the preceding claims, wherein the image sensor (30) is centred with respect to the axis of incidence along which the incident beam (12) reaching the object (3) propagates.

4. Device according to any one of the preceding claims, wherein the diameter of the aperture (23) is smaller than 2 cm.

5. Device according to any one of the preceding claims, wherein the screen (20) has a curved shape and/or wherein the screen (20) is translucent.

6. Device according to any one of the preceding claims, wherein the screen (20) comprises a light guide for conveying light between the first face (20₁) and the second face (20₂) and/or wherein the screen comprises a plurality of optical fibres extending between the first face (20₁) and the second face (20₂).

7. Device according to any one of the preceding claims, wherein the holder (6) is movable with respect to the screen (20), the distance (d) between the holder and the screen being adjustable.

8. Device according to any one of the preceding claims, wherein the incident beam (12) propagates between the screen (20) and the object (3) about an axis called the axis of incidence (Z), the device comprising what is called an annular reflector (18), lying around the axis of incidence, between the sample (2) and the screen (20), the annular reflector being able to reflect some of the radiation (14) back-scattered toward the screen (20).

9. Method for observing an object (3) present in a sample (2), the sample lying facing a screen (20) comprising a first face (20₁), the method comprising the following steps:
a) illuminating the object (3) using an incident light beam (12) emitted by a light source (10), the screen lying between the light source and the object (3), the incident light beam (12) propagating to the object through an aperture (23) in the screen;
b) exposing a first face (20₁) of the screen to light radiation (14) back-scattered by the sample, so as to form, on the first face (20₁), an image, called a scattergram, representative of said back-scattered radiation (14), the screen comprising a second face (20₂), so that the scattergram formed on the first face (20₁) appears on the second face (20₂);
c) acquiring an image of the scattergram formed on the screen with an image sensor (30), the screen lying between the image sensor (30) and the object (3), the image sensor (30) being coupled to the second face (20₂) by a focusing optic (25);
the method being **characterized in that**:
- the light beam emitted by the light source is directed toward the object (3), through the aperture (23), by a reflective element (13);
- the reflective element (13) is placed between the object (3) and the image sensor (30);
- the reflective element (13) absorbs at least 30% of the back-scattered radiation (14) emitted by the object;
- and/or the reflective element (13) is fastened to a holder (17), the holder absorbing at least 30% of the back-scattered radiation (14) emitted by the object.

10. Method according to Claim 9, comprising, following step c), a step of adjusting the distance (d) between the sample (2) and the screen (20) depending on the image acquired by the image sensor (30), steps a) to c) being repeated after the adjustment of said distance.

11. Method according to either of Claims 9 and 10, comprising a step d) of characterizing the object (3) on the basis of the image acquired by the image sensor.

12. Method according to Claim 11, wherein step d) comprises:
- determining characteristics of the image;
- identifying the object using said characteristics and calibration characteristics established by implementing steps a) to c) of the method on a standard sample.

13. Method according to any one of Claims 9 to 12, wherein the object comprises a microorganism.
